# EUROPEAN PATENT SPECIFICATION

(11) **EP 1 924 198 B1**
(45) Date of publication and mention of the grant of the patent: **03.04.2019**
(21) Application number: 06814538.2
(22) Date of filing: 12.09.2006
(51) Int. Cl.: A61B 5/05

(54) **APPARATUS FOR IMAGE GUIDED ACCURACY VERIFICATION**
VORRICHTUNG ZUR BILDGELENKTEN PRÄZISIONSPRÜFUNG
DISPOSITIF DE VÉRIFICATION DE PRÉCISION UTILISANT UN GUIDAGE PAR IMAGES

(30) Priority: 13.09.2005 US 224028; 25.04.2006 US 410143
(43) Date of publication of application: 28.05.2008
(60) Divisional application: 19153753.9
(73) Proprietor: Veran Medical Technologies, Inc., St. Louis, Missouri 63110 (US)
(72) Inventor: LYON, Torsten, M., Nashville, TN 37212 (US); HOLSING, Troy, L., Nashville, TN 37212 (US); EDWARDS, Jerome, R., Nashville, TN 37212 (US); LEE, Christopher, B., Nashville, TN 37212 (US); AUSTILL, Evan, Jr., Nashville, TN 37212 (US)
(74) Representative: Gill Jennings & Every LLP
(86) International application number: PCT/US2006/035548
(87) International publication number: WO 2007/033206

(56) References cited:
- EP-A2- 1 319 368
- WO-A1-94/24933
- WO-A2-99/27839
- US-A- 6 016 439
- US-A- 6 122 541
- US-A1- 2004 167 393
- US-B1- 6 381 485

## Description

### Background

The invention relates generally to a medical device and particularly to an apparatus and method associated with image guided medical procedures.

Image guided surgery (IGS), also known as image guided intervention (IGI), enhances a physician's ability to locate instruments within anatomy during a medical procedure. IGS can include 2-dimensional (2-D) and 3-dimensional (3-D) applications.

Existing imaging modalities can capture the movement of dynamic anatomy. Such modalities include electrocardiogram (ECG)-gated or respiratory-gated magnetic resonance imaging (MRI) devices, ECG-gated or respiratory-gated computer tomography (CT) devices, and cinematography (CINE) fluoroscopy. The dynamic imaging modalities can capture the movement of anatomy over a periodic cycle of that movement by sampling the anatomy at several instants during its characteristic movement and then creating a set of image frames or volumes. Such images can be used to help a physician navigate a medical instrument to the desired location on the anatomical body during a medical procedure performed on the anatomical body at a later time.

Typical image-guided medical systems require manual user input to identify a pre-procedural image that corresponds to the same position and orientation of an anatomical body during a medical procedure. These manual operations can lead to greater errors and reduced efficiency in image-guided procedures.

A need exists for an apparatus that can be used with such imaging devices to capture pre-procedural images of a targeted anatomical body and use those images intra-procedurally to guide a physician to the correct location of the anatomical body during a medical procedure.

A need also exists for a method and apparatus that can automatically identify pre-procedural images of a targeted anatomical body that can be used to help a physician navigate a medical instrument to a selected location on the anatomical body during a range of motion of the anatomical body.

US6122541A discloses a headband for frameless stereotactic registration in which markers and ancillary markers are positioned on a headband worn by a patient wherein the positions of the markers and ancillary markers are fixed relative to one another. US6381485B discloses a system for registration of human anatomy for electromagnetic localisation wherein separate sensors and fiducial markers are placed, in known and fixed relation to one another, on a patient's head. WO9927839A discloses a surgical positioning system in which radio-opaque markers are disposed on a patient's body and subsequently included in images obtained using a medical imaging device and by an optical tracking system during treatment of the patient.

### Summary of the Invention

In a first aspect, the invention consists an apparatus, comprising: a first marker coupled to a garment at a first location, a second marker coupled to the garment at a second location, the garment configured to be coupled to a dynamic body in a fixed position relative to the dynamic body, characterised in that the first and second markers change in orientation relative to each other during a path of motion of the dynamic body and wherein the first and second markers are radio-opaque; a first localization element is coupled to the garment proximate the location of the first marker; and a second localization element is coupled to the garment proximate the location of the second marker, the first localization element and the second localization element comprising electromagnetic coils and each being coupled to a receiving device and configured to simultaneously send to the receiving device position data associated with a plurality of positions in three-dimensional space of the first localization element and position data associated with a plurality of positions in three-dimensional space of the second localization element during the path of motion of the dynamic body, wherein the first and second localization elements change in orientation relative to each other during the path of motion of the dynamic body, wherein the receiving device determines a vector distance between the position of the first localization element and the position of the second localization element based on the position data for each of a plurality of instants of time during the path of motion of the dynamic body.

In a second aspect, the invention consists in a processor-readable medium storing code representing instructions to cause a processor to perform a process, the process comprising: receiving during a first time interval image data associated with a plurality of images of a dynamic body, the plurality of images including an indication of a position of a first marker coupled to the dynamic body and a position of a second marker coupled to the dynamic body, the first marker coupled to the dynamic body at a first location, the second marker coupled to the dynamic body at a second location, the first time interval associated with a first path of motion of the dynamic body, wherein the first and second markers change in orientation relative to each other during the first path of motion of the dynamic body and wherein the first and second markers are radio-opaque; receiving during a second time interval after the first time interval data associated with a position of a first localization element relative to the dynamic body, and data associated with a position of a second localization element relative to the dynamic body, the first localization element coupled to the dynamic body proximate the first location, the second localization element coupled to the dynamic body proximate the second location, the second time interval associated with a second path of motion of the dynamic body, wherein the first and second localization elements change in orientation relative to each other during the second path of motion of the dynamic body and wherein the first and second localization elements are electromagnetic coils; determining a vector distance between the position of the first localisation element and the position of the second localisation element based on the position data for each of a plurality of instance of time during the path of motion of the dynamic body; and automatically identifying during the second time interval an image from the plurality of images associated with a position of the first marker that is substantially the same as the position of the first localization element relative to the dynamic body and a position of the second marker that is substantially the same as the position of the second localization element relative to the dynamic body.

The present invention is described with reference to the accompanying drawings.
FIG. 1 is a schematic illustration of various devices that can be used with a method according to an embodiment of the invention.
FIG. 2 is a schematic illustration of various devices that can be used with a method according to an embodiment of the invention.
FIG. 3 is a schematic illustrating vector distances on an apparatus according to an embodiment of the invention.
FIG. 4 is a schematic illustrating vector distances from a localization device according to an embodiment of the invention.
FIG. 5 is a schematic illustrating vector distances from image data according to an embodiment of the invention.
FIG. 6 is a front perspective view of an apparatus according to an embodiment of the invention.
FIG. 7 is a graphical representation illustrating the function of an apparatus according to an embodiment of the invention.
FIG. 8 is a flowchart illustrating a method according to an embodiment of the invention.
FIG. 9 is a schematic illustration of various devices that can be used with a method according to an embodiment of the invention.
FIG. 10 is a schematic illustration of various devices that can be used with a method according to an embodiment of the invention.
FIG. 11 is a schematic illustration of the flow of information during an automatic segmentation process.
FIG. 12 is a schematic illustration of an example of voxels of a connected-component in a 3-D volume according to an embodiment of the invention.
FIG. 13 is a schematic illustration of an example of voxels of a connected-component in a 3-D volume according to an alternative embodiment of the invention.
FIG. 14 is a flowchart illustrating a method according to an embodiment of the invention.

### Detailed Description

An apparatus according to an embodiment of the invention includes a garment and two or more markers coupled to the garment. The apparatus can also include two or more localization elements coupled to the garment proximate the markers. The apparatus is configured to be coupled to a dynamic body, such as selected dynamic anatomy of a patient. Dynamic anatomy can be, for example, any anatomy that moves during its normal function (e.g., the heart, lungs, kidneys, liver and blood vessels). A processor, such as a computer, is configured to receive image data associated with the dynamic body taken during a pre-surgical or pre-procedural first time interval. The image data can include an indication of a position of each of the markers for multiple instants in time during the first time interval. The processor can also receive position data associated with the localization elements during a second time interval in which a surgical procedure or other medical procedure is being performed. The processor can use the position data received from the localization elements to determine a distance between the elements for a given instant in time during the second time interval. The processor can also use the image data to determine the distance between the markers for a given instant in time during the first time interval. The processor can then find a match between an image where the distance between the markers at a given instant in time during the first time interval is the same as the distance between the elements associated with those markers at a given instant in time during the medical procedure, or second time interval.

A physician or other healthcare professional can use the images selected by the processor during a medical procedure performed during the second time interval. For example, when a medical procedure is performed on a targeted anatomy of a patient, such as a heart, the physician may not be able to utilize an imaging device during the medical procedure to guide him to the targeted area within the patient. A garment according to an embodiment of the invention can be positioned or coupled to the patient proximate the targeted anatomy prior to the medical procedure, and pre-procedural images can be taken of the targeted area during a first time interval. Markers or fiducials coupled to the garment can be viewed with the image data, which can include an indication of the position of the markers during a given path of motion of the targeted anatomy (e.g., the heart) during the first time interval. Such motion can be due, for example, to inspiration (i.e., inhaling) and expiration (i.e., exhaling) of the patient, or due to the heart beating. During a medical procedure, performed during a second time interval, such as a procedure on a heart, the processor receives data from the localization elements associated with a position of the elements at a given instant in time during the medical procedure (or second time interval). The distance between selected pairs of markers can be determined from the image data and the distance between corresponding selected pairs of localization elements can be determined based on the element data for given instants in time.

Because the localization elements are coupled to the garment proximate the location of the markers, the distance between a selected pair of elements can be used to determine an intra-procedural distance between the pair of corresponding markers to which the localization elements are coupled. An image from the pre-procedural image data taken during the first time interval can then be selected where the distance between the pair of selected markers in that image corresponds with or closely approximates the same distance determined using the localization elements at a given instant in time during the second time interval. This process can be done continuously during the medical procedure, producing simulated real-time, intra-procedural images illustrating the orientation and shape of the targeted anatomy as a catheter or similar structure is navigated to the targeted anatomy. Thus, during the medical procedure, the physician can view selected image(s) of the targeted anatomy that correspond to and simulate real-time movement of the anatomy. In addition, during a medical procedure being performed during the second time interval, such as navigating a catheter to a targeted anatomy, the location(s) of an electromagnetic coil coupled to the catheter during the second time interval can be superimposed on an image of a catheter. The superimposed image(s) of the catheter can then be superimposed on the selected image(s) from the first time interval, providing simulated real time images of the catheter location relative to the targeted anatomy. This process and other related methods are described in US2005/038337 entitled Methods, Apparatuses, and Systems Useful in Conducting Image Guided Interventions.

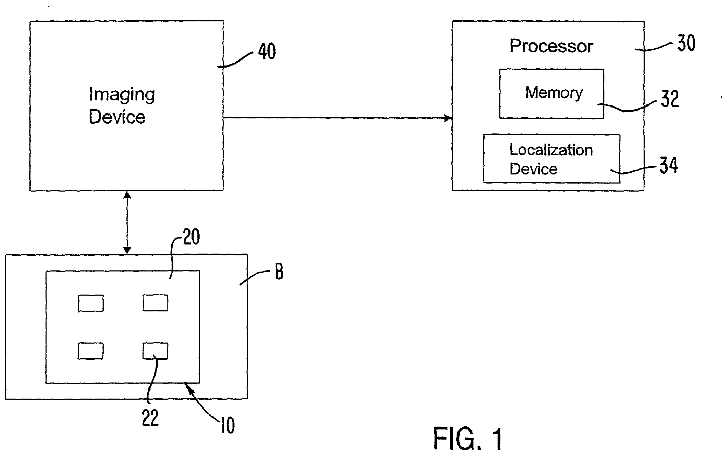

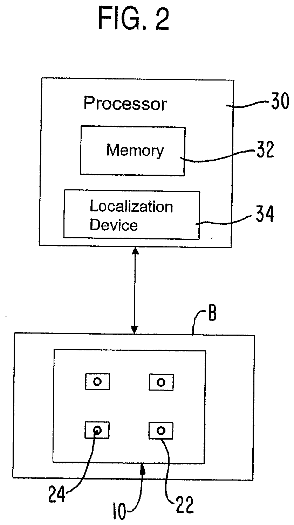

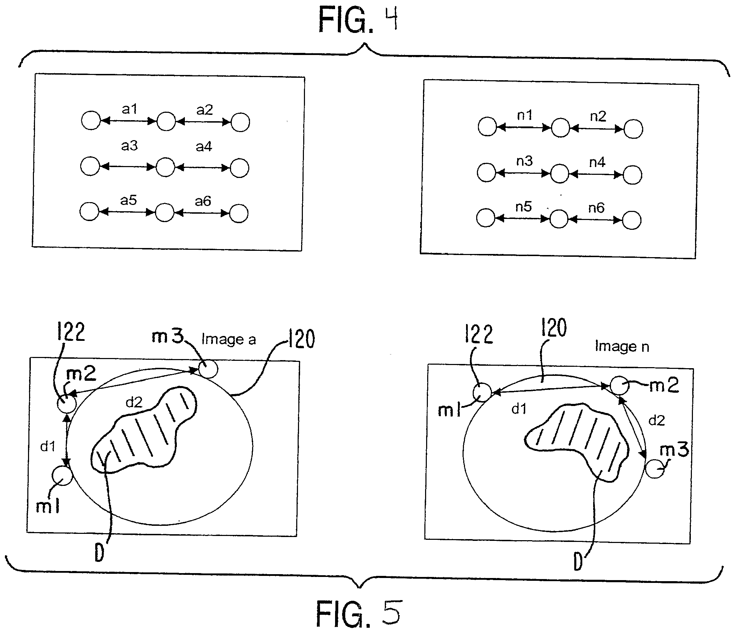

FIGS. 1 and 2 are schematic illustrations of devices that can be used to perform various procedures described herein. As shown in FIG. 1, an apparatus 10 includes a garment 20. The garment 20 can be coupled to a dynamic body B. The dynamic body B can be, for example, a selected dynamic portion of the anatomy of a patient. The garment 20 can be a variety of different shapes and sizes. For example, in one embodiment the garment 20 is a tubular or sleeve configuration (see FIG. 5) and can fit, for example, around the torso of a patient, or around the upper chest surrounding, for example, the patient's heart. The garment 20 can be a continuous tubular configuration or a partial tubular configuration. For example, the garment 20 may be substantially planar prior to coupling to the dynamic body and then wrapped around the dynamic body and coupled to the dynamic body using an attachment, such as straps, hook and pile fastener, snaps, or any other suitable coupling method. In the case of a continuous tubular shape, the garment 20 may be held in position on the dynamic body through friction fit, or due to the garment being stretchable such that it conforms to the dynamic body. In another embodiment, the garment 20 is substantially planar, such as in the form of a patch that can be disposed at a variety of locations on a patient's body. Such a garment 20 can be coupled to the dynamic body with adhesive, straps, hook and pile, snaps, or any other suitable coupling method.

In some embodiments, the garment 20 is configured as a shirt to be worn by a patient. In some embodiments, the garment 20 is configured to be worn similar to a pair of pants. In still other embodiments, a garment is configured as an undergarment to be worn by a patient. For example, a garment can be configured as an undergarment to be worn on the upper torso of the patient (e.g., a brassiere). These configurations may allow, for example, placement of markers at varying angles relative to the targeted anatomy of the patient.

The garment 20 can be constructed with a variety of different materials, such as fabric, plastic, and rubber and can be flexible, stretchable and/or rigid. In some embodiments, the garment 20 is configured to constrict movement of the dynamic body B. For example, the garment 20 can be constructed in a tubular configuration with a stretchable material that when coupled to the patient's body, constricts at least a portion of the patient's movement through inhaling and exhaling or movement caused by the heart beating.

Two or more markers or fiducials 22 are coupled to the garment 20 at selected locations as shown in FIG. 1. The markers 22 are constructed of a material that can be viewed on an image, such as an X-ray. The markers 22 can be, for example, radiopaque, and can be coupled to the garment 20 using any known methods of coupling such devices. FIGS. 1 and 2 illustrate the apparatus 10 having four markers 22, but any number of two or more markers can be used.

An imaging device 40 can be used to take images of the dynamic body B while the garment 20 is coupled to the dynamic body B, pre-procedurally during a first time interval. As stated above, the markers 22 are visible on the images and can provide an indication of a position of each of the markers 22 during the first time interval. The position of the markers 22 at given instants in time through a path of motion of the dynamic body B can be illustrated with the images. The imaging device 40 can be, for example, a computed tomography (CT) device (e.g., respiratory-gated CT device, ECG-gated CT device), a magnetic resonance imaging (MRI) device (e.g., respiratory-gated MRI device, ECG-gated MRI device), an X-ray device, or any other suitable medical imaging device. In one embodiment, the imaging device 40 is a computed tomography - positron emission tomography device that produces a fused computed tomography - positron emission tomography image dataset. The imaging device 40 can be in communication with a processor 30 and send, transfer, copy and/or provide image data taken during the first time interval associated with the dynamic body B to the processor 30.

The processor 30 includes a processor-readable medium storing code representing instructions to cause the processor 30 to perform a process. The processor 30 can be, for example, a commercially available personal computer, or a less complex computing or processing device that is dedicated to performing one or more specific tasks. For example, the processor 30 can be a terminal dedicated to providing an interactive graphical user interface (GUI). The processor 30, according to one or more embodiments of the invention, can be a commercially available microprocessor. Alternatively, the processor 30 can be an application-specific integrated circuit (ASIC) or a combination of ASICs, which are designed to achieve one or more specific functions, or enable one or more specific devices or applications. In yet another embodiment, the processor 30 can be an analog or digital circuit, or a combination of multiple circuits.

The processor 30 can include a memory component 32. The memory component 32 can include one or more types of memory. For example, the memory component 32 can include a read only memory (ROM) component and a random access memory (RAM) component. The memory component can also include other types of memory that are suitable for storing data in a form retrievable by the processor 30. For example, electronically programmable read only memory (EPROM), erasable electronically programmable read only memory (EEPROM), flash memory, as well as other suitable forms of memory can be included within the memory component. The processor 30 can also include a variety of other components, such as for example, coprocessors, graphic processors, etc., depending upon the desired functionality of the code.

The processor 30 can store data in the memory component 32 or retrieve data previously stored in the memory component 32. The components of the processor 30 can communicate with devices external to the processor 30 by way of an input/output (I/O) component (not shown). According to one or more embodiments of the invention, the I/O component can include a variety of suitable communication interfaces. For example, the I/O component can include, for example, wired connections, such as standard serial ports, parallel ports, universal serial bus (USB) ports, S-video ports, local area network (LAN) ports, small computer system interface (SCCI) ports, and so forth. Additionally, the I/O component can include, for example, wireless connections, such as infrared ports, optical ports, Bluetooth® wireless ports, wireless LAN ports, or the like.

The processor 30 can be connected to a network, which may be any form of interconnecting network including an intranet, such as a local or wide area network, or an extranet, such as the World Wide Web or the Internet. The network can be physically implemented on a wireless or wired network, on leased or dedicated lines, including a virtual private network (VPN).

As stated above, the processor 30 can receive image data from the imaging device 40. The processor 30 can identify the position of selected markers 22 within the image data or voxel space using various segmentation techniques, such as Hounsfield unit thresholding, convolution, connected component, or other combinatory image processing and segmentation techniques. The processor 30 can determine a distance and direction between the position of any two markers 22 during multiple instants in time during the first time interval, and store the image data, as well as the position and distance data, within the memory component 32. Multiple images can be produced providing a visual image at multiple instants in time through the path of motion of the dynamic body. The processor 30 can also include a receiving device or localization device 34, which is described in more detail below.

As shown in FIG. 2, two or more localization elements 24 are coupled to the garment 20 proximate the locations of the markers 22 for use during a medical procedure to be performed during a second time interval. The localization elements 24 can be, for example, electromagnetic coils, infrared light emitting diodes, and/or optical passive reflective markers. The markers 22 can include plastic or non-ferrous fixtures or dovetails or other suitable connectors used to couple the localization elements 24 to the markers 22. A medical procedure can then be performed with the garment 20 coupled to the dynamic body B at the same location as during the first time interval when the pre-procedural images were taken. During the medical procedure, the localization elements 24 are in communication or coupled to the localization device 34 included within processor 30. The localization device 34 can be, for example, an analog to digital converter that measures voltages induced onto localization coils in the field; creates a digital voltage reading; and maps that voltage reading to a metric positional measurement based on a characterized volume of voltages to millimeters from a fixed field emitter. Position data associated with the elements 24 can be transmitted or sent to the localization device 34 continuously during the medical procedure during the second time interval. Thus, the position of the localization elements 24 can be captured at given instants in time during the second time interval. Because the localization elements 24 are coupled to the garment 20 proximate the markers 22, the localization device 34 can use the position data of the elements 24 to deduce coordinates or positions associated with the markers 22 intra-procedurally during the second time interval. The distance between one or more selected pairs of localization elements 24 (and corresponding markers 22) can then be determined and various algorithms can be used to analyze and compare the distance between selected elements 24 at given instants in time, to the distances between and orientation among corresponding markers 22 observed in the pre-operative images.

An image can then be selected from the pre-operative images taken during the first time interval that indicates a distance between corresponding markers 22 at a given instant in time, that most closely approximates or matches the distance between the selected elements 24. The process of comparing the distances is described in more detail below. Thus, the apparatus 10 and processor 30 can be used to provide images corresponding to the actual movement of the targeted anatomy during the medical procedure being performed during the second time interval. The images illustrate the orientation and shape of the targeted anatomy during a path of motion of the anatomy, for example, during inhaling and exhaling.

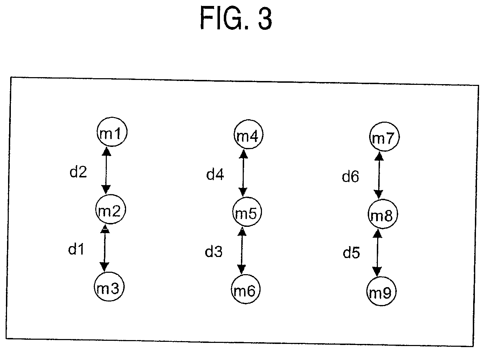

FIG. 3 illustrates an example set of distances or vectors d1 through d6 between a set of markers 122, labeled m1 through m9 that are disposed at spaced locations on a garment 120. As described above, pre-procedure images can be taken of a dynamic body for which the garment 120 is to be coupled during a first time interval. The distances between the markers can be determined for multiple instants in time through the path of motion of the dynamic body. Then, during a medical procedure, performed during a second time interval, localization elements (not shown in FIG. 3) coupled proximate to the location of markers 122 can provide position data for the elements to a localization device (not shown in FIG. 3). The localization device can use the position data to determine distances or vectors between the elements for multiple instants in time during the medical procedure or second time interval.

FIG. 4 shows an example of distance or vector data from the localization device. Vectors a1 through a6 represent distance data for one instant in time and vectors n1 through n6 for another instant in time, during a time interval from a to n. As previously described, the vector data can be used to select an image from the pre-procedural images that includes distances between the markers m1 through m9 that correspond to or closely approximate the distances a1 through a6 for time a, for example, between the localization elements. The same process can be performed for the vectors n1 through n6 captured during time n.

One method of selecting the appropriate image from the pre-procedural images is to execute an algorithm that can sum all of the distances a1 through a6 and then search for and match this sum to an image containing a sum of all of the distances d1 through d6 obtained pre-procedurally from the image data that is equal to the sum of the distances a1 through a6. When the difference between these sums is equal to zero, the relative position and orientation of the anatomy or dynamic body D during the medical procedure will substantially match the position and orientation of the anatomy in the particular image. The image associated with distances d1 through d6 that match or closely approximate the distances a1 through a6 can then be selected and displayed. For example, FIG. 5 illustrates examples of pre-procedural images, Image a and Image n, of a dynamic body D that correspond to the distances a1 through a6 and n1 through n6, respectively. An example of an algorithm for determining a match is as follows:

| | |
|---|---|
| Does | ∑ aᵢ = ∑ dᵢ (i =1 to 6 in this example) OR |
| Does | ∑ (aᵢ - dᵢ) = 0 (i =1 to 6 in this example). |

If yes to either of these, then the image is a match to the vector or distance data obtained during the medical procedure.

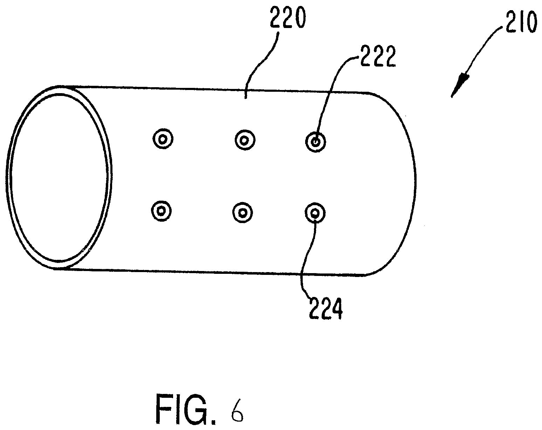

FIG. 6 illustrates an apparatus 210 according to an embodiment of the invention. The apparatus 210 includes a tubular shaped garment 220 that can be constructed with a flexible and/or stretchable material. The garment 220 can be positioned over a portion of a patient's body, such as around the upper or lower torso of the patient. The stretchability of the garment 220 allows the garment 220 to at least partially constrict some of the movement of the portion of the body for which it is coupled. The apparatus 210 further includes multiple markers or fiducials 222 coupled to the garment 220 at spaced locations. A plurality of localization elements 224 are removably coupled proximate to the locations of markers 222, such that during a first time interval as described above, images can be taken without the elements 224 being coupled to the garment 220. The localization elements need not be removably coupled. For example, the elements can be fixedly coupled to the garment. In addition, the elements can be coupled to the garment during the pre-procedure imaging.

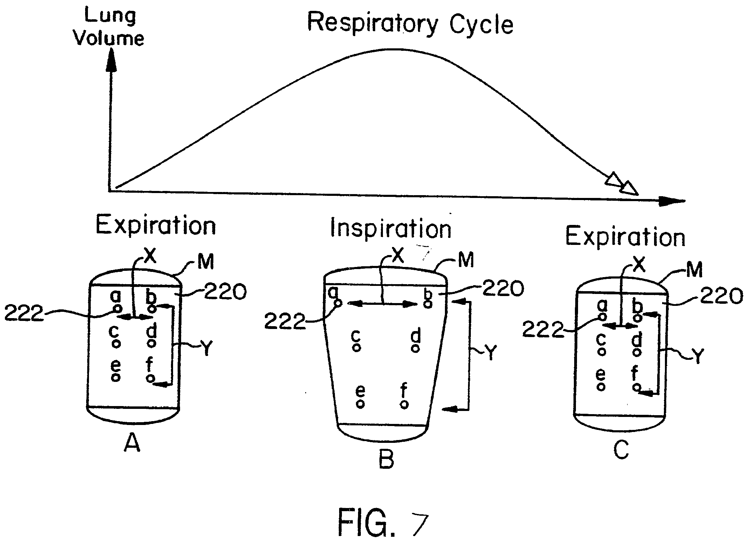

FIG. 7 is a graphical illustration indicating how the apparatus 210 (shown without localization elements 224) can move and change orientation and shape during movement of a dynamic body, such as a mammalian body M. The graph is one example of how the lung volume can change during inhalation (inspiration) and exhalation (expiration) of the mammalian body M. The corresponding changes in shape and orientation of the apparatus 210 during inhalation and exhalation are also illustrated. The six markers 222 shown in FIG. 6 are labeled a, b, c, d, e, and f. As described above, images of the apparatus 110 can be taken during a first time interval. The images can include an indication of relative position of each of the markers 222, that is the markers 222 are visible in the images, and the position of each marker 222 can then be observed over a period of time. A distance between any two markers 222 can then be determined for any given instant of time during the first time interval. For example, a distance X between markers a and b is illustrated, and a distance Y between markers b and f is illustrated. These distances can be determined for any given instant in time during the first time interval from an associated image that illustrates the position and orientation of the markers 222. As illustrated, during expiration of the mammalian body M at times indicated as A and C, the distance X is smaller than during inspiration of the mammalian body M, at the time indicated as B. Likewise, the distance Y is greater during inspiration than during expiration. The distance between any pair of markers 222 can be determined and used in the processes described herein. Thus, the above embodiments are merely examples of possible pair selections. For example, a distance between a position of marker e and a position of marker b may be determined. In addition, multiple pairs or only one pair may be selected for a given procedure.

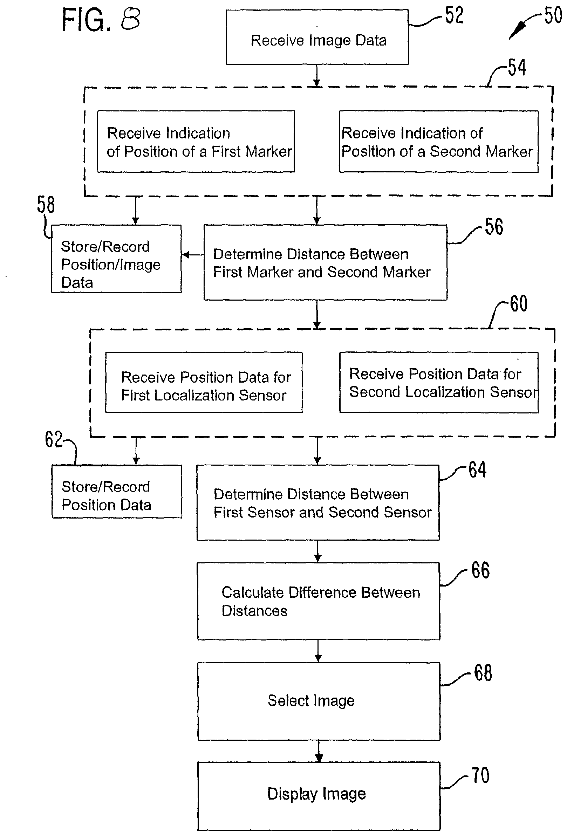

FIG. 8 is a flowchart illustrating a method according to an embodiment of the invention. A method 50 includes at step 52 receiving image data during a pre-procedural or first time interval. As discussed above, images are taken of a dynamic body using an appropriate imaging modality (e.g., CT Scan, MRI, etc.). The image data is associated with one or more images taken of a garment (as described herein) coupled to a dynamic body, where the garment includes two or more markers coupled thereto. In other words, the image data of the dynamic body is correlated with image data related to the garment. The one or more images can be taken using a variety of different imaging modalities as described previously. The image data can include an indication of a position of a first marker and an indication of a position of a second marker, as illustrated at step 54. The image data can include position data for multiple positions of the markers during a range or path of motion of the dynamic body over a selected time interval. As described above, the image data can include position data associated with multiple markers, however, only two are described here for simplicity. A distance between the position of the first marker and the position of the second marker can be determined for multiple instants in time during the first time interval, at step 56. As also described above, the determination can include determining the distance based on the observable distance between the markers on a given image. The image data, including all of the images received during the first time interval, the position, and the distance data can be stored in a memory and/or recorded at step 58.

Then at step 60, during a second time interval, while performing a medical procedure on the patient with the garment positioned on the patient at substantially the same location, position data can be received for a first localization element and a second localization element. The localization elements can be coupled to the garment proximate the locations of the markers, such that the position data associated with the elements can be used to determine the relative position of the markers in real-time during the medical procedure. The position data of the elements can be stored and/or recorded at step 62.

A distance between the first and second localization elements can be determined at step 64. Although only two localization elements are described, as with the markers, position data associated with more than two localization elements can be received and the distances between the additional elements can be determined.

The next step is to determine which image from the one or more images taken during the first time interval represents the relative position and/or orientation of the dynamic body at a given instant in time during the second time interval or during the medical procedure. To determine this, at step 66, the distance between the positions of the first and second localization elements at a given instant in time during the second time interval are compared to the distance(s) determined in step 56 between the positions of the first and second markers obtained with the image data during the first time interval.

An image can be selected from the first time interval that best represents the same position and orientation of the dynamic body at a given instant in time during the medical procedure. To do this, the difference between the distance between a given pair of localization elements during the second time interval is used to select the image that contains the same distance between the same given pair of markers from the image data received during the first time interval. This can be accomplished, for example, by executing an algorithm to perform the calculations. When there are multiple pairs of markers and localization elements, the algorithm can sum the distances between all of the selected pairs of elements for a given instant in time during the second time interval and sum the distances between all of the associated selected pairs of markers for each instant in time during the first time interval when the pre-procedural image data was received.

When an image is found that provides the sum of distances for the selected pairs of markers that is substantially the same as the sum of the distances between the localization elements during the second time interval, then that image is selected at step 68. The selected image can then be displayed at step 70. The physician can then observe the image during the medical procedure on a targeted portion of the dynamic body. Thus, during the medical procedure, the above process can be continuously executed such that multiple images are displayed and images corresponding to real-time positions of the dynamic body can be viewed.

In another embodiment, a method includes capturing images of a dynamic body during a path of motion of the dynamic body pre-procedurally (also referred to herein as "first time interval"). The images can be used to assist a physician in navigating a medical instrument to a desired location on the dynamic body during a medical procedure performed at a later time (also referred to herein as "second time interval"). The method uses a system configured to automatically perform segmentation, correlation and registration between data obtained in "model space" or "image space" (position data taken pre-procedurally) and data obtained in "physical space" (position data obtained during a later medical procedure).

Specifically, an apparatus is configured to be coupled to a selected dynamic body, such as selected dynamic anatomy of a patient. Dynamic anatomy can be, for example, any portion of the body associated with anatomy that moves during its normal function (e.g., the heart, lungs, kidneys, liver and vasculature). The apparatus can include, for example, two or more markers configured to be coupled to a patient and two or more localization elements configured to be coupled to the patient proximate the markers. In other embodiments, the apparatus can include, for example, a garment (as described above) configured to be coupled to a patient, two or more markers coupled to the garment, and two or more localization elements coupled to the garment at a location proximate the markers.

A processor, such as a computer, is configured to receive the pre-procedural image data associated with the dynamic body taken during a pre-surgical or pre-procedural first time interval. The image data can include an indication of a position of each of the markers for multiple instants in time during the first time interval. The processor can also receive position data associated with the localization elements during a second time interval in which a surgical procedure or other medical procedure is being performed. The processor can use the position data received from the localization elements and the position data received from the images to automatically identify an image from the pre-procedural images where the position of the markers at a given instant in time during the pre-procedural imaging is substantially the same as the position of the localization elements corresponding to those markers, at a given instant of time during the later medical procedure.

A physician or other healthcare professional can use the images that were automatically identified by the processor during a medical procedure performed during the second time interval, such as, for example, an image-guided medical procedure involving temporal registration and gated navigation. For example, when a medical procedure is performed on a targeted anatomy of a patient, such as a heart, the physician may not be able to utilize an imaging device during the medical procedure to guide him to the targeted area within the patient. Markers or fiducials can be positioned or coupled to the patient proximate the targeted anatomy prior to the medical procedure, and pre-procedural images can be taken of the targeted area during a first time interval. The markers or fiducials can be viewed within the image data, which can include an indication of the position of the markers during a given path of motion of the targeted anatomy (e.g., the heart) during the first time interval. Such motion can be due, for example, to inspiration (i.e., inhaling) and expiration (i.e., exhaling) of the patient, or due to the heart beating. During a medical procedure performed during a second time interval, such as a procedure on a heart, with the markers coupled to the patient at the same location/position as during the first time interval, the processor receives data from the localization elements associated with a position of the localization elements at a given instant in time during the medical procedure (or second time interval).

Because the markers are positioned at the same location on the patient relative to the dynamic body during both the first time interval and the second time interval, and the localization elements are coupled to the patient proximate the location of the markers, a correlation can be made between the position data in image space and the position data in physical space. For example, a position of the markers at an instant in time during the pre-procedural imaging corresponds to a specific position and orientation of the dynamic body at an instant in time during the path of motion of the dynamic body as viewed m the image data. When the medical procedure is performed during the second time interval, a position of the localization elements likewise corresponds to a specific positioning of the dynamic body at an instant in time during the path of motion of the dynamic body. Although the marker-localization element combinations can move relative to each other, for example, as the dynamic anatomy moves, the markers are in a fixed position relative to the patient during both the first time interval and the second time interval. As stated above, the localization elements are coupled to the patient proximate the markers, thus, when the position of the localization elements (identified during the medical procedure) is substantially the same as the position of the markers (identified in the image space), the image corresponding to that position of the markers is representative of the position of the dynamic body for that instant during the medical procedure.

An automatic segmentation-correlation-registration process can be performed after the image dataset is imported into the processor and the localization elements are connected to the processor. Once performed, the correlation does not change during the course of the procedure and the model space marker positions provide a baseline position for the temporal registration. After the segmentation-correlation and baseline registration has been computed, the localization element locations are sampled automatically and continuously to determine when the dynamic body is at or near the position at which the images were acquired. The affine rigid-body transformation is computed automatically and continuously until a temporal gating threshold is exceeded, indicating that the dynamic body is no longer near the same configuration as where the images were acquired. The automatic process produces simulated real-time, intra-procedural images illustrating the orientation and shape of the targeted anatomy as a catheter or similar structure is navigated to the targeted anatomy. Thus, during the medical procedure, the physician can view selected image(s) of the targeted anatomy that correspond to and simulate real-time movement of the anatomy.

In addition, during a medical procedure being performed during the second time interval, such as navigating an instrument, such as a catheter or needle to a targeted anatomy, the location(s) of an electromagnetic coil coupled to the instrumentation during the second time interval can be superimposed on an image of the instrumentation. The superimposed image(s) of the instrument can then be superimposed on the selected image(s) from the first time interval, providing simulated real time imaging of the instrument location relative to the targeted anatomy. This process and other related methods are described in US2005/038337.

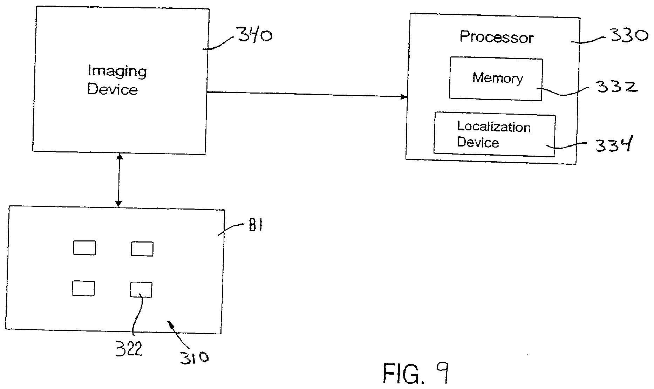

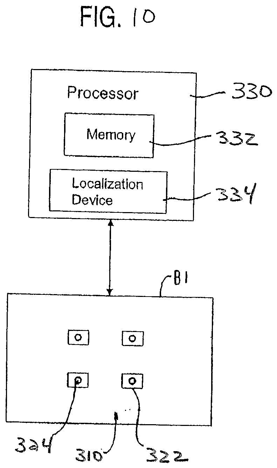

FIGS. 9 and 10 are schematic illustrations of devices that can be used to perform various procedures described herein. An apparatus 310 includes two or more markers or fiducials 322 coupled to a dynamic body B1 at selected locations, as shown in FIG. 9. The dynamic body B1 can be, for example, a selected dynamic portion of the anatomy of a patient. The markers 322 are constructed of a material that can be viewed on an image, such as an X-ray. The markers 322 can be, for example, radiopaque, and can be coupled to the dynamic body B1 using known methods of coupling such devices to a patient, such as with adhesive, straps, etc. FIGS. 9 and 10 illustrate the apparatus 310 having four markers 322, but any number of two or more markers can be used.

An imaging device 340 can be used to take images of the dynamic body B1 while the markers 322 are coupled to the dynamic body B, pre-procedurally during a first time interval. As stated above, the markers 322 are visible on the images and can provide an indication of a position of each of the markers 322 during the first time interval. The position of the markers 322 at given instants in time through a path of motion of the dynamic body B1 can be illustrated with the images. The imaging device 340 can be, for example, any of the embodiments described above for imaging device 40, , or any other suitable medical imaging device. The imaging device 340 can be in communication with a processor 330 and send, transfer, copy and/or provide image data taken during the first time interval associated with the dynamic body B1 to the processor 330.

The processor 330 includes a processor-readable medium storing code representing instructions to cause the processor 330 to perform a process. The processor 330 can be, for example, any embodiment or configuration for a processor as described above for processor 30, and can perform the same functions. The processor 330 can also include a memory component 332 that

can include, for example, any of the embodiments or configurations described above for memory 32. Thus, the processor 330 and the memory 332 can perform substantially the same functions as processor 30 and memory 32.

The processor 330 can also be connected to a network, which may be any form of interconnecting network including an intranet, such as a local or wide area network, or an extranet, such as the World Wide Web or the Internet. The network can be physically implemented on a wireless or wired network, on leased or dedicated lines, including a virtual private network (VPN).

As stated above, the processor 330 can receive image data (also referred to herein as "image dataset") from the imaging device 340. The processor 330 can identify the position of selected markers 322 within the image data or voxel space using various segmentation techniques, such as Hounsfield unit thresholding, convolution, connected component, or other combinatory image processing and segmentation techniques. The processor 330 can determine a distance and direction between the position of any two markers 322 during multiple instants in time during the first time interval, and store the image data, as well as the position and distance data, within the memory component 332. Multiple images can be produced providing a visual image at multiple instants in time through the path of motion of the dynamic body. The processor 330 can also include a receiving device or localization device 334, which is described in more detail below.

As shown in FIG. 10, during a second time interval, two or more localization elements 324 are coupled to the markers 322 for use during a medical procedure to be performed during the second time interval. The localization elements 324 are coupled to the patient adjacent the markers 322. The localization elements 324 can be, for example, electromagnetic coils, infrared light emitting diodes, and/or optical passive reflective markers. The markers 322 can include plastic or non-ferrous fixtures or dovetails or other suitable connectors used to couple the localization elements 324 to the markers 322. A medical procedure can then be performed with the markers 322 coupled to the dynamic body B1 at the same location as during the first time interval when the pre-procedural images were taken. During the medical procedure, the localization elements 324 are in communication or coupled to the localization device 334 included within processor 330. The localization device 334 can be, for example, an analog to digital converter that measures voltages induced onto localization coils in the field; creates a digital voltage reading; and maps that voltage reading to a metric positional measurement based on a characterized volume of voltages to millimeters from a fixed field emitter. Position data associated with the localization elements 324 can be transmitted or sent to the localization device 334 continuously during the medical procedure during the second time interval. Thus, the position of the localization elements 324 can be captured at given instants in time during the second time interval.

The image dataset, the position data for the markers from the first time interval ("model space") and the position data for the localization elements during the second time interval ("physical space") can be used to perform an automatic segmentation, correlation and registration between the data in the model space and the data in the physical space. The result of the analysis is to provide a physician with images that represent the position of a dynamic body during the second time interval when the physician is performing a medical procedure on the dynamic body. The processor 330 can be configured to perform the automatic segmentation-correlation-registration process as described in more detail below.

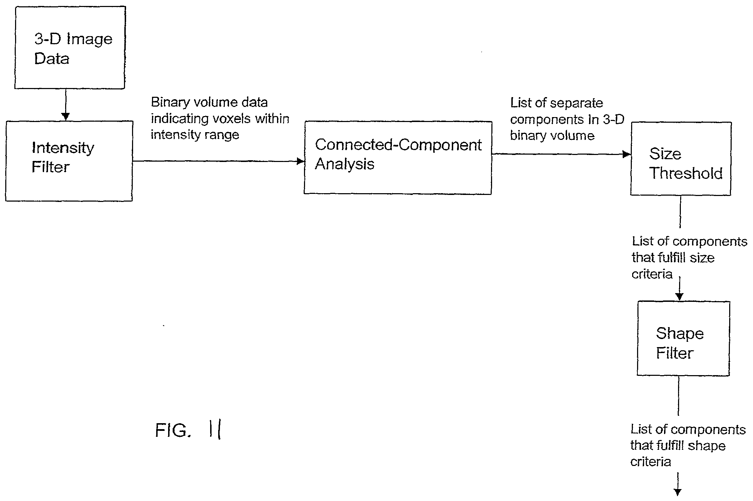

To identify actual position data associated with the markers 322 within the image dataset, the processor 330 can perform an automated segmentation procedure. Segmentation is the process of identifying reference points in the 3-D image dataset. The purpose of the segmentation is to automatically locate potential "landmarks" in the dataset that indicate a location where a marker 322 may be located. Segmentation can be performed in a variety of different manners. For example, a segmentation process can include, intensity filtering, connectivity analysis, and size and shape filtering to identify candidate sensor (e.g., marker) locations, or model space (also referred to herein as "image space") coordinates of the marker 320 candidates. In some example embodiments, the intensity filtering applies domain knowledge to threshold the 3-D image dataset to select only those image values that fall within a designated intensity range that contains the reference points. For example, reference markers can be designated to appear in CT scans with Hounsfield units higher than the anatomical structures within the 3-D image. An example output from an intensity filtering process can include a 3-D binary volume with non-zero entries indicating voxels (i.e., a 3-D data point) with an intensity that falls within the range of values that characterize an image marker, as illustrated in FIG. 11. FIG. 11 is a schematic illustration of the flow of information during one example of an automatic segmentation process.

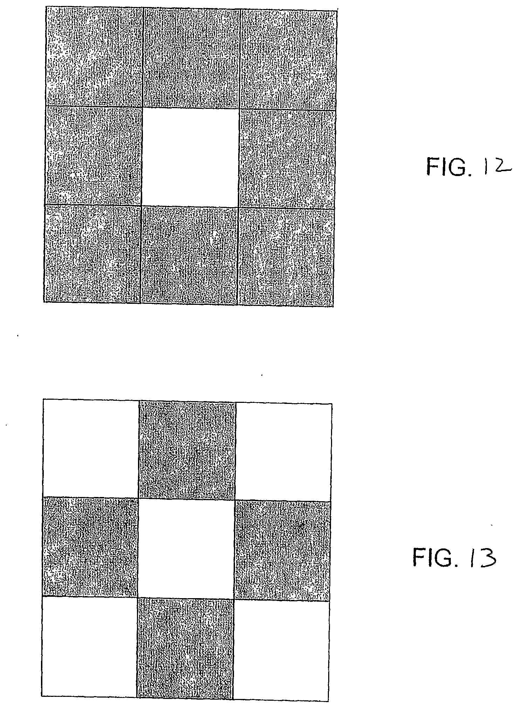

After filtering the image values based on intensity, a connectivity analysis can be performed. A connectivity analysis can use the output from the intensity filtering to analyze the potential candidates identified in the 3-D image dataset to identify, for example, "connected-components." A connected-component, as used here, is a continuous 3-D region in the 3-D volume (i.e., image dataset) that is connected via adjacent voxels that are in contact with one another. Examples of voxels of connected-components are illustrated in FIGS. 12 and 13. FIG. 12 illustrates a connected-component having 8 connected voxel elements (indicated by the shaded boxes), and FIG. 13 illustrates a connected component having 4 connected voxel elements (indicated by the shaded boxes). From the identified connected-components, information about the connected regions, such as the location of each voxel element, the geometric center, and the volume and bounding perimeter dimensions can be identified. An example output of a connectivity analysis can include, for example, a list of each separate connected-component in the 3-D binary volume, and can be used in the next step in the segmentation process.

Next, in some embodiments, the output from the connectivity analysis, which includes the identified connected-components, can be filtered based on size and shape criteria during a size threshold analysis. First, knowledge about the size of the reference markers can be used to identify and discard any connected-components that are too small or too large to be valid markers. A list of connected-components that fulfill the size criteria can then be evaluated based on the shape of the components during a shape-filtering analysis. Knowledge about the shape of the reference markers can be used to discard any components that do not match the known shape of the reference markers. For example, if the markers are known to be cylindrical, then the connected component shape can be analyzed to determine if the ratio between the major axis and the minor axis is within a set criteria. The output from this step in this example process includes, for example, a list of connected-components that fulfill the shape criteria. Other analysis can be performed depending on the particular marker configuration, such as, for example, checking whether the connected-component shape is symmetric about a centroid of the connected-component.

After the segmentation process is performed, an automatic correlation process can be performed. Correlation as used here is the process of correctly matching reference points between the image or model space and the physical space. Correctly matching the reference points aids in accurately computing the registration between the data in the image space and the data in the physical space without user interaction. The correlation process determines where each of the localization elements is positioned in the model images. Correct correlation is required to compute an affine transform between model space and physical space. The apparatuses and methods described herein enable the process to be automated with minimal user intervention. Automatic correlation results in an understanding of the location of the markers in image space and physical space, as well as the corresponding labeling/identification of each marker in each space.

Because there are a large number of possible solutions, computations of all possible combinations can result in long computation times. According to an embodiment of the invention, the processor 330 can be configured to compute the correlation between the image space and the physical space at a much faster rate (e.g., 2 seconds on a 1.5 GHz G4 Macintosh computer).

Because the number of localization element positions in the physical space is typically smaller than the number of identified marker positions in the model space, a guess at a correlation can be made for three localization element points in physical space. An affine transform registration is then computed between the selected positions of the localization elements 324 in physical space and the model space. The computed registration is then used to transform the remaining localization element positions to model space and determine if any markers exist at the projected locations. A brute force iteration is made in groups of 3 as just described. When projecting the remaining points from physical space to model space to test the correlation guess, a test can be performed for the existence of a marker in model space within a settable threshold 3-D distance. If present, a 3-D error can be computed and the correlation resulting in the lowest error can be noted and recorded. This technique discards points in model space that do not have a corresponding point in physical space (i.e., false positives in the list of marker positions determined during segmentation).

Because the number of localization element positions is relatively low, it can be fairly computationally inexpensive to perform the iterative process described above to search all possible correlation combinations. The process is implemented such that the affine transform used to compute rigid body registration between the model space and the physical space for each 3-point correlation is abstract, and the actual implementation can be defined and determined at runtime. It is possible to improve the speed of the process by stopping the solution search iterations if a solution is identified that meets the specified criteria. For example, when computing the error for a correlation guess, the projection loop-and-fail for the correlation guess can be reduced if any single reference point in the physical space fails to map to a point in model space within a specified error threshold. Each potential correlation combination is evaluated by one or more criteria to determine the correlation between segmented markers in model space and physical localization element locations. Examples of evaluation criteria include computing the transformation using three points, and then projecting the remaining physical points to model space as described previously. Other examples include incorporating coil orientation information between the segmented markers and 5- or 6-degrees of freedom (DOF) localization elements, or applying externally available information, such as requiring the user to attach the localization elements in a certain configuration. This correlation technique can account for physical localization elements being in a slightly different relative position than the model space markers since the localization elements process can be performed when the localization elements are anywhere in the periodic cycle of the dynamic body.

After the correlation process, the processor 330 can perform an automatic registration process. The process of registration tracks temporal movement of the dynamic body via the movement of the markers 322, and when temporally valid, computes the transformation between the physical space and the model space.

A measure of a temporal position is referred to herein as a "cost-function." An automatic registration algorithm uses abstract objects so that the cost-function can be defined/determined at runtime. For example, one possible cost function is the average distance between reference points (e.g., positions of localization elements 324). Cost-functions can compute a temporal measure for a group of reference points independent of the space in which the points are known since the measure is based upon landmark positions relative to each other. Once the correlation is established, the localization element locations in physical space can be periodically evaluated using a cost-function to determine when the dynamic body most closely matches the point in the periodic phase of the first time interval (image acquisition). Examples of cost-functions can include: average distance between markers; max/min axis ratio of bounding ellipsoid; and a ratio between minimum and maximum 3D distance between markers. The cost-function can be, for example, determined in patient model space to ensure that moving the patient and/or localizing machinery will not affect the outcome/solution/computation.

A cost-function can be used to establish a measure of the marker positions within the plurality of images during the first time interval. The same cost-function can then be applied continuously to the correlated localization element positions during the second time interval. When the cost-function indicates that the positions of the localization elements in the second time interval have the same relative positions as the marker positions in the first time interval, then the dynamic body can be identified as being at the same temporal point along the path of motion as the first time interval. During the time that the cost-function indicates that the dynamic body is at the same temporal point along the path of motion as the first time interval, then the automatically correlated markers from the first time interval and localization elements from the second time interval can be used to automatically compute a registration. When the cost-function indicates that the registration is valid, then the position and navigational path of a medical instrument can be displayed on a computer screen superimposed onto images of the dynamic body acquired during the first time interval.

After performing the automated segmentation and correlation processes, a list of position data for the localization elements 324 in image space is obtained. This represents the position of the markers 322, and therefore the position of the dynamic body B1 when the image dataset was acquired. This information is used as the "temporal reference" for the image dataset and represents the nominal reference point position for the dataset. For multiple images acquired at different points in the patient temporal cycle (e.g., at inspiration and expiration of the respiratory cycle), the segmentation-correlation process can be repeated and a temporal reference position can be determined for each image.

Once the temporal reference is established for each image dataset, a registration filter can be used to compare the position of the localization elements 124 in the physical space to the temporal reference location for each image dataset. If the positions of the localization elements 324 are sufficiently close to the temporal reference for a dataset (i.e., the image dataset), then the dataset can be used for navigation for that temporal moment by computing the affine transformation between the physical space and model space. The transformation is then used to project information such as reformatted images, segmentations, informatics, etc. The threshold that determines how close the physical configuration must be to the locations in the image dataset can be modified at runtime to allow the sensitivity or temporal resolution to be modified.

Through the automatic registration process, the relative marker positions at the time of the 3-D scan can be determined. This acquisition of relative marker position allows the point in the respiratory cycle at which the scan was acquired to be determined and navigation gated to that same point in the cycle during a subsequent medical procedure. The resulting registration is relative to the markers affixed to the patient, which allows the patient to be repositioned relative to the scan gantry, table position, and/or localization machinery without invalidating the registration, as long as the markers remain in a fixed position relative to the patient.

As stated previously, the automatic segmentation-correlation-registration process can be performed using an apparatus that includes a garment, such as the garments 20, 120, and 220 described above. Such an apparatus can be used with the systems and methods described in this embodiment to perform the same automatic-segmentation-registration processes described above, except in such an embodiment, the markers and localization elements are coupled to the patient through the use of a garment. All other devices described with reference to FIGS. 9 and 10 can be used in such an embodiment to perform the same automatic segmentation-correlation-registration processes as described above.

A garment can be positioned over a portion of a patient's body (proximate dynamic anatomy), such as around the upper or lower torso of the patient at a fixed location relative to the patient during both a first time period, in which images are taken of the dynamic anatomy (model or image space), and during a second time period, in which a medical procedure is being performed on the dynamic anatomy (physical space). The stretchability of the garment allows the garment to at least partially constrict some of the movement of the portion of the body for which it is coupled. Markers can be coupled to the garment at a fixed location on the garment, thus the markers are also coupled to the patient at a fixed location relative to the dynamic anatomy during both the first time period and the second time period.

Referring back to FIG. 7, although FIG. 7 is being described with reference to an embodiment including a garment, an embodiment that does not include a garment can be similarly described. The six markers 222 shown in FIG. 7 are labeled a, b, c, d, e, and f. As described above, images of the dynamic anatomy with an apparatus, such as apparatus 210, coupled thereto can be taken during a first time interval. The images can include an indication of relative position of each of the markers 222, that is the markers 222 are visible in the images, and the position of each marker 222 can then be identified over a period of time. As illustrated, during expiration of the mammalian body M at times indicated as A and C, a distance X between markers a and b is smaller than during inspiration of the mammalian body M, at the time indicated as B. Likewise, a distance Y between markers b and f is greater during inspiration than during expiration.

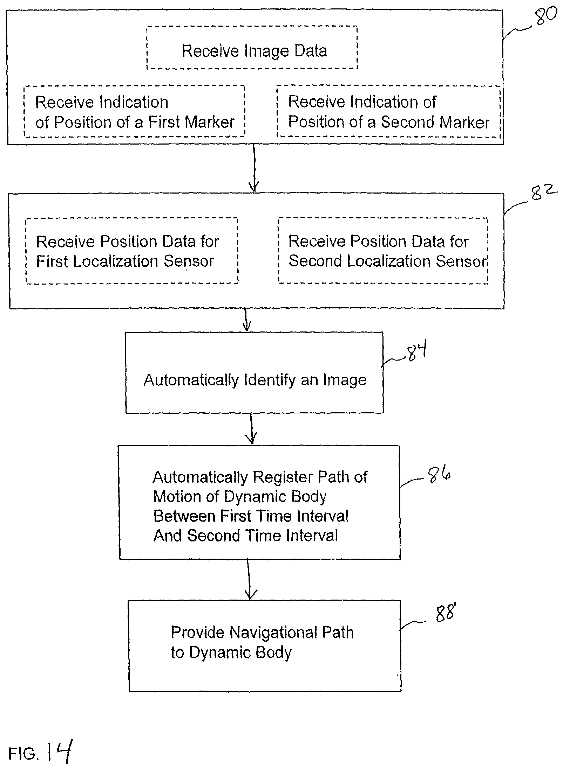

FIG. 14 is a flowchart illustrating a method according to another embodiment of the invention. A method includes at step 80 receiving during a first time interval image data associated with a plurality of images of a dynamic body. The plurality of images include an indication of a position of a first marker on a garment coupled to the dynamic body and a position of a second marker on the garment coupled to the dynamic body. The first marker is coupled to the garment at a first location and the second marker is coupled to the garment at a second location. The first time interval is associated with a path of motion of the dynamic body. At step 82, data associated with a position of a first localization element relative to the dynamic body is received, and data associated with a position of a second localization element relative to the dynamic body is received during a second time interval after the first time interval. The first localization element is coupled to the garment at the first location, and the second localization element is coupled to the garment at the second location. The second time interval is associated with a path of motion of the dynamic body and the garment is coupled to the dynamic body in a fixed position relative to the dynamic body during both the first time interval and the second time interval.

During the second time interval, an image from the plurality of images associated with a position of the first marker that is substantially the same as the position of the first localization element relative to the dynamic body and a position of the second marker that is substantially the same as the position of the second localization element relative to the dynamic body are automatically identified at step 84. The automatic identification can be based on an appearance of the markers within the identified image. The automatic identification can also include identifying a position of a third localization element, and projecting that position on to the image data set and determining whether a third marker exists in an image from the image data set at the position of the third localization element. The automatic identification can also include correlating a position of the first localization element during the second time interval with the position of the first marker in the plurality of images. At step 86, the path of motion of the dynamic body is automatically registered during the second time interval is automatically registered with the path of motion of the dynamic body during the first time interval. The automatic registering in step 86 can include identifying at least one temporal reference within the plurality of images and identifying whether the at least one temporal reference is associated with at least one of the first marker or the second marker providing a navigational path for a medical instrument to be directed based on the identified image.

At step 88, a navigational path is provided for a medical instrument to be directed based on the identified image. A physician can use the navigational path to guide a medical instrument to the dynamic body while performing a medical procedure on the dynamic body during the second time interval.

### Conclusion

While various embodiments of the invention have been described above, it should be understood that they have been presented by way of example only, and not limitation. Thus, the breadth and scope of the invention should not be limited by any of the above-described embodiments, but should be defined only in accordance with the following claims and their equivalents.

The previous description of the embodiments is provided to enable any person skilled in the art to make or use the invention. While the invention has been particularly shown and described with reference to embodiments thereof, it will be understood by those skilled in art that various changes in form and details may be made therein without departing from the scope of the invention. For example, the garment, markers and localization elements can be constructed from any suitable material, and can be a variety of different shapes and sizes, not necessarily specifically illustrated, while still remaining within the scope of the invention.

While a relatively small number of markers are discussed, the system is scalable and the use of any number of markers is contemplated. For example, a garment may include between 2 and 20 markers, 10-50 markers, etc. Additionally, variations in the automated processes can be used to achieve the same, or substantially the same, desired results.

## Claims

1. An apparatus (10), comprising:
a first marker (22) coupled to a garment (20) at a first location, a second marker (22) coupled to the garment (20) at a second location, the garment configured to be coupled to a dynamic body (B) in a fixed position relative to the dynamic body (B),
**characterised in that** the first and second markers change in orientation relative to each other during a path of motion of the dynamic body and wherein the first and second markers are radio-opaque;
a first localization element (24) is coupled to the garment proximate the location of the first marker; and
a second localization element (24) is coupled to the garment proximate the location of the second marker, the first localization element and the second localization element comprising electromagnetic coils and each being coupled to a receiving device (34) and configured to simultaneously send to the receiving device position data associated with a plurality of positions in three-dimensional space of the first localization element and position data associated with a plurality of positions in three-dimensional space of the second localization element during the path of motion of the dynamic body, wherein the first and second localization elements change in orientation relative to each other during the path of motion of the dynamic body (B),
wherein the receiving device determines a distance (d1 - d6) between the position of the first localization element and the position of the second localization element based on the position data for each of a plurality of instants of time during the path of motion of the dynamic body.

2. The apparatus of claim 1, wherein the garment (20) is substantially planar.

3. The apparatus of claim 1, wherein the garment (20) has a sleeve configuration.

4. The apparatus of claim 1, wherein the garment (20) is configured to be coupled to the upper torso of a patient.

5. The apparatus of claim 1, wherein the garment (20) is configured to at least partially constrict movement of the dynamic body (B).

6. The apparatus of claim 1, wherein the garment (20) is configured to be coupled to the dynamic body (B) using at least one of an adhesive, one or more straps, one or more hook and pile fasteners, or one or more snaps.

7. The apparatus of claim 1, wherein the garment (20) is in the form of a patch configured to be disposed on a patient's body.

8. A processor-readable medium storing code representing instructions to cause a processor (30) to perform a process, the process comprising:
receiving during a first time interval image data associated with a plurality of images of a dynamic body (B), the plurality of images including an indication of a position of a first marker (22) coupled to the dynamic body and a position of a second marker (22) coupled to the dynamic body, the first marker coupled to the dynamic body at a first location, the second marker coupled to the dynamic body at a second location, the first time interval associated with a first path of motion of the dynamic body, wherein the first and second markers change in orientation relative to each other during the first path of motion of the dynamic body and wherein the first and second markers are radio-opaque;
receiving during a second time interval after the first time interval data associated with a position of a first localization element (24) relative to the dynamic body, and data associated with a position of a second localization element (24) relative to the dynamic body, the first localization element coupled to the dynamic body proximate the first location, the second localization element coupled to the dynamic body proximate the second location, the second time interval associated with a second path of motion of the dynamic body, wherein the first and second localization elements change in orientation relative to each other during the second path of motion of the dynamic body and wherein the first and second localization elements are electromagnetic coils;
determining a distance (D1-D6) between the position of the first localisation element and the position of the second localisation element based on the position data for each of a plurality of instance of time during the path of motion of the dynamic body; and
automatically identifying during the second time interval an image from the plurality of images associated with a position of the first marker that is substantially the same as the position of the first localization element relative to the dynamic body and a position of the second marker that is substantially the same as the position of the second localization element relative to the dynamic body.

9. The processor-readable medium of claim 8, wherein the first marker (22) is coupled to a garment (20), the garment being coupled to the dynamic body (B) and the second marker (22) being coupled to the garment, the first marker coupled to the garment at the first location, the second marker coupled to the garment at the second location;
the first localization element (24) coupled to the garment at the first location, the second localization element (24) coupled to the garment at the second location.

10. The processor-readable medium of claims 8 or 9, wherein the automatically identifying includes identifying a position of the first marker (22) based on an appearance of the first marker within the identified image and identifying a position of the second marker (22) based on an appearance of the second marker within the identified image.

11. The processor-readable medium of claims 8 or 9, the code also representing instructions to cause the processor (30) to further perform the process of:
receiving during the second time interval data associated with a position of a third localization element (24) relative to the dynamic body (B), the third localization element coupled to the dynamic body;
projecting the position of the third localization element based on the data associated with the plurality of images; and
determining that a third marker (22) either one of exists or does not exist in an image from the plurality of images from the first time interval at substantially the same position as the third localization element.

12. The processor-readable medium of claims 8 or 9, wherein the automatically identifying includes correlating the position of the first localization element (24) during the second time interval with the position of the first marker (22) within an image from the plurality of images.

13. The processor-readable medium of claims 8 or 9, the code also representing instructions to cause the processor (30) to further perform the process of:
automatically registering the path of motion of the dynamic body (B) during the second time interval with the path of motion of the dynamic body during the first time interval based on the automatically identifying.

14. The processor-readable medium of claims 8 or 9,
wherein the automatically identifying includes determining a measurement between the position of the first marker (22) and the position of the second marker (22) associated with the plurality of images during the first time interval using a selected cost-function;
applying the selected cost-function to the position of the first localization element (24) and the position of the second localization element (24) during the second time interval;
determining whether the position of the first localization element and the position of the second localization element during the second time interval is substantially the same as the position of the first marker and the position of the second marker, respectively, during the first time interval;
automatically computing a registration between the positions of the first and second markers, and the positions of the first and second localizations elements; and superimposing a position of a medical instrument onto an image display of the dynamic body (B) from the first time interval.

15. The processor-readable medium of claim 9, wherein the garment (20) is coupled to the dynamic body (B) in a fixed position relative to the dynamic body during both the first time interval and the second time interval.

16. The processor-readable medium of claims 8 or 9, the code also representing instructions to cause the processor (30) to further perform the process of:
providing a navigational path for a medical instrument to be directed based on the identified image.

17. The processor-readable medium of claims 8 or 9, wherein the first marker (22) and the second marker (22) are coupled to the dynamic body (B) in a fixed position relative to the dynamic body, but can move relative to each other when the dynamic body moves through a path of motion, the first localization element (24) and the second localization element (24) are coupled to the dynamic body at a fixed position relative to the dynamic body, but can move relative to each other when the dynamic body moves through a path of motion.

18. The processor-readable medium of claims 8 or 9, wherein the receiving an indication of a position of a first marker (22) and an indication of a position of the second marker (22) includes receiving a voxel coordinate of the first marker at the first location and a voxel coordinate of the second marker at the second location.

19. The processor-readable medium method of claim 9, wherein the garment (20) has a substantially planar configuration.

20. The processor-readable medium of claims 8 or 9, wherein the receiving image data includes receiving image data from a computed tomography device.

21. The processor-readable medium of claims 8 or 9, wherein the receiving image data includes receiving image data from a computed tomography-positron emission tomography device that produces a fused computed tomography-positron emission tomography image dataset.

22. The processor-readable medium of claims 8 or 9, wherein the receiving image data includes receiving image data from a magnetic resonance imaging device.

23. The processor-readable medium of claims 8 or 9, the code also representing instructions to cause the processor (30) to further perform the process of:
receiving the data associated with a position of the first localization element (24) and the data associated with a position of the second localization element (24) continuously during the second time interval and recording the data associated with a position of the first localization element and the data associated with a position of the second localization element continuously during the second time interval.

24. The processor-readable medium of claim 9, the code also representing instructions to cause the processor (30) to further perform the process of:
receiving during the first time interval an indication of a position of a third marker (22) on the garment and a position of a fourth marker (22) on the garment (20), the third marker coupled to the garment at a third location and the fourth marker coupled to the garment at a fourth location, wherein the third and fourth markers change in orientation relative to each other during the first time interval;
determining a distance between the position of the third marker and the position of the fourth marker;
receiving during the second time interval data associated with a position of a third localization element (24) coupled to the garment at the third location and data associated with a position of a fourth localization element (24) coupled to the garment at the fourth location, wherein the third and fourth localization elements change in orientation relative to each other during the second time interval;
determining a distance between the third localization element and the fourth localization element based on the data associated with the position of the third localization element and the position of the second localization element; and
calculating whether the distance between the third marker and the fourth marker during the first time interval is different from the distance between the third localization element and the fourth localization element during the second time interval.

25. The processor-readable medium of claim 9, wherein the garment (20) is configured to at least partially constrict movement of the dynamic body (B).

26. The processor-readable medium of claims 8 or 9, wherein the automatically identifying includes automatically segmenting the data associated with the plurality of images from the first time interval to identify a position of the first marker (22) and a position of the second marker (22).

27. The processor-readable medium of claims 8 or 9, wherein the automatically identifying includes automatically correlating the data associated with the position of the first marker (22) and the position of the second marker (22) from the first time interval with the data associated with the position of the first localization element (24) and the position of the second localization element (24).

28. The processor-readable medium of claims 8 or 9, wherein the automatically identifying includes automatically registering the path of motion of the dynamic body (B) during the first time interval with the path of motion of the dynamic body during the second time interval.

29. The processor-readable medium of claim 8 or 9, wherein the plurality of images include a plurality of 3-D images of the dynamic body (B) during a path of motion of the dynamic body.

## Patentansprüche

1. Eine Vorrichtung (10), die Folgendes beinhaltet:
eine erste Markierung (22), die an einem ersten Ort mit einem Kleidungsstück (20) gekoppelt ist, eine zweite Markierung (22), die an einem zweiten Ort mit dem Kleidungsstück (20) gekoppelt ist, wobei das Kleidungsstück dazu ausgelegt ist, in einer relativ zu dem dynamischen Körper (B) fixierten Position mit einem dynamischem Körper (B) gekoppelt zu werden,
**dadurch gekennzeichnet, dass** die erste und zweite Markierung sich im Verlauf einer Bewegungsbahn des dynamischen Körpers in ihrer Ausrichtung zueinander ändern und wobei die erste und zweite Markierung röntgendicht sind;
ein erstes Lokalisierungselement (24) in der Nähe des Orts der ersten Markierung mit dem Kleidungsstück gekoppelt ist; und
ein zweites Lokalisierungselement (24) in der Nähe des Orts der zweiten Markierung mit dem Kleidungsstück gekoppelt ist, wobei das erste Lokalisierungselement und das zweite Lokalisierungselement elektromagnetische Spulen beinhalten und jeweils mit einem Empfangsgerät (34) gekoppelt sind und dazu ausgelegt sind, gleichzeitig Positionsdaten, die mit einer Vielzahl von Positionen im dreidimensionalen Raum des ersten Lokalisierungselements assoziiert sind, und Positionsdaten, die mit einer Vielzahl von Positionen im dreidimensionalen Raum des zweiten Lokalisierungselements assoziiert sind, im Verlauf der Bewegungsbahn des dynamischen Körpers an das Empfangsgerät zu senden, wobei das erste und zweite Lokalisierungselement sich im Verlauf der Bewegungsbahn des dynamischen Körpers (B) in ihrer Ausrichtung zueinander ändern,
wobei das Empfangsgerät einen Abstand (d1- d6) zwischen der Position des ersten Lokalisierungselements und der Position des zweiten Lokalisierungselements, basierend auf den Positionsdaten für jeden von einer Vielzahl von Zeitpunkten im Verlauf der Bewegungsbahn des dynamischen Körpers, bestimmt.

2. Die Vorrichtung gemäß Anspruch 1, wobei das Kleidungsstück (20) im Wesentlichen planar ist.

3. Die Vorrichtung gemäß Anspruch 1, wobei das Kleidungsstück (20) eine Ärmelkonfiguration aufweist.

4. Die Vorrichtung gemäß Anspruch 1, wobei das Kleidungsstück (20) dazu ausgelegt ist, mit dem oberen Torso eines Patienten gekoppelt zu werden.

5. Die Vorrichtung gemäß Anspruch 1, wobei das Kleidungsstück (20) dazu ausgelegt ist, die Bewegung des dynamischen Körpers (B) mindestens teilweise einzuschränken.

6. Die Vorrichtung gemäß Anspruch 1, wobei das Kleidungsstück (20) dazu ausgelegt ist, unter Verwendung von mindestens einem von einem Kleber, einem oder mehreren Gurten, einem oder mehreren Klettverschlüssen oder einem oder mehreren Druckknöpfen mit dem dynamischen Körper (B) gekoppelt zu werden.

7. Die Vorrichtung gemäß Anspruch 1, wobei das Kleidungsstück (20) die Form eines Pflasters aufweist, das zum Anordnen auf dem Körper eines Patienten ausgelegt ist.

8. Ein prozessorlesbares Medium, das Code speichert, der Anweisungen darstellt, um einen Prozessor (30) zum Durchführen eines Verfahrens zu veranlassen, wobei das Verfahren Folgendes beinhaltet:
Empfangen, während eines ersten Zeitintervalls, von mit einer Vielzahl von Bildern eines dynamischen Körpers (B) assoziierten Bilddaten, wobei die Vielzahl von Bildern einen Indikator einer Position einer ersten Markierung (22), die mit dem dynamischen Körper gekoppelt ist, und einer Position einer zweiten Markierung (22), die mit dem dynamischen Körper gekoppelt ist, umfasst, wobei die erste Markierung an einem ersten Ort mit dem dynamischen Körper gekoppelt ist, wobei die zweite Markierung an einem zweiten Ort mit dem dynamischen Körper gekoppelt ist, wobei das erste Zeitintervall mit einer ersten Bewegungsbahn des dynamischen Körpers assoziiert ist, wobei die erste und zweite Markierung sich im Verlauf der ersten Bewegungsbahn des dynamischen Körpers in ihrer Ausrichtung zueinander ändern und wobei die erste und zweite Markierung röntgendicht sind;
Empfangen, während eines zweiten Zeitintervalls nach dem ersten Zeitintervall, von Daten, die mit einer Position eines ersten Lokalisierungselements (24) relativ zu dem dynamischen Körper assoziiert sind, und von Daten, die mit einer Position eines zweiten Lokalisierungselements (24) relativ zu dem dynamischen Körper assoziiert sind, wobei das erste Lokalisierungselement in der Nähe des ersten Orts mit dem dynamischen Körper gekoppelt ist, wobei das zweite Lokalisierungselement in der Nähe des zweiten Orts mit dem dynamischen Körper gekoppelt ist, wobei das zweite Zeitintervall mit einer zweiten Bewegungsbahn des dynamischen Körpers assoziiert ist, wobei das erste und zweite Lokalisierungselement sich während der zweiten Bewegungsbahn des dynamischen Körpers in ihrer Ausrichtung zueinander ändern und wobei das erste und zweite Lokalisierungselement elektromagnetische Spulen sind;
Bestimmen eines Abstands (D1-D6) zwischen der Position des ersten Lokalisierungselements und der Position des zweiten Lokalisierungselements, basierend auf den Positionsdaten für jeden von einer Vielzahl von Zeitpunkten im Verlauf der Bewegungsbahn des dynamischen Körpers; und
automatisches Identifizieren, während des zweiten Zeitintervalls, eines Bilds aus der Vielzahl von Bildern, die mit einer Position der ersten Markierung, die im Wesentlichen gleich ist wie die Position des ersten Lokalisierungselements relativ zu dem dynamischen Körper, und einer Position der zweiten Markierung, die im Wesentlichen gleich ist wie die Position des zweiten Lokalisierungselements relativ zu dem dynamischen Körper, assoziiert sind.

9. Das prozessorlesbare Medium gemäß Anspruch 8, wobei die erste Markierung (22) mit einem Kleidungsstück (20) gekoppelt ist, wobei das Kleidungsstück mit dem dynamischen Körper gekoppelt (B) ist, und die zweite Markierung (22) mit dem Kleidungsstück gekoppelt ist, wobei die erste Markierung an dem ersten Ort mit dem Kleidungsstück gekoppelt ist, wobei die zweite Markierung an dem zweiten Ort mit dem Kleidungsstück gekoppelt ist;
wobei das erste Lokalisierungselement (24) an dem ersten Ort mit dem Kleidungsstück gekoppelt ist, wobei das zweite Lokalisierungselement (24) an dem zweiten Ort mit dem Kleidungsstück gekoppelt ist.

10. Das prozessorlesbare Medium gemäß Ansprüchen 8 oder 9, wobei das automatische Identifizieren das Identifizieren einer Position der ersten Markierung (22), basierend auf einem Erscheinen der ersten Markierung innerhalb des identifizierten Bilds, und das Identifizieren einer Position der zweiten Markierung (22), basierend auf einem Erscheinen der zweiten Markierung innerhalb des identifizierten Bilds, umfasst.

11. Das prozessorlesbare Medium gemäß Ansprüchen 8 oder 9, wobei der Code auch Anweisungen darstellt, um den Prozessor (30) zu veranlassen, des Weiteren folgende Verfahren durchzuführen:
Empfangen, während des zweiten Zeitintervalls, von Daten, die mit einer Position eines dritten Lokalisierungselements (24) relativ zu dem dynamischen Körper (B) assoziiert sind, wobei das dritte Lokalisierungselement mit dem dynamischen Körper gekoppelt ist;
Projizieren der Position des dritten Lokalisierungselements, basierend auf den mit der Vielzahl von Bildern assoziierten Daten; und
Bestimmen, dass eine dritte Markierung (22) in einem Bild aus der Vielzahl von Bildern aus dem ersten Zeitintervall an der im Wesentlichen gleichen Position wie das dritte Lokalisierungselement entweder existiert oder nicht existiert.

12. Das prozessorlesbare Medium gemäß Ansprüchen 8 oder 9, wobei das automatische Identifizieren das Korrelieren der Position des ersten Lokalisierungselements (24) während des zweiten Zeitintervalls mit der Position der ersten Markierung (22) innerhalb eines Bilds aus der Vielzahl von Bildern umfasst.

13. Das prozessorlesbare Medium gemäß Ansprüchen 8 oder 9, wobei der Code auch Anweisungen darstellt, um den Prozessor (30) zu veranlassen, des Weiteren folgende Verfahren durchzuführen:
automatisches Registrieren der Bewegungsbahn des dynamischen Körpers (B) während des zweiten Zeitintervalls mit der Bewegungsbahn des dynamischen Körpers während des ersten Zeitintervalls, basierend auf dem automatischen Identifizieren.

14. Das prozessorlesbare Medium gemäß Ansprüchen 8 oder 9,
wobei das automatische Identifizieren das Bestimmen einer Messung zwischen der Position der ersten Markierung (22) und der Position der zweiten Markierung (22), die mit der Vielzahl von Bildern während des ersten Zeitintervalls assoziiert sind, unter Verwendung einer ausgewählten Kostenfunktion, umfasst;
Anwenden der ausgewählten Kostenfunktion auf die Position des ersten Lokalisierungselements (24) und die Position des zweiten Lokalisierungselements (24) während des zweiten Zeitintervalls;
Bestimmen, ob die Position des ersten Lokalisierungselements und die Position des zweiten Lokalisierungselements während des zweiten Zeitintervalls im Wesentlichen gleich ist wie die Position der ersten Markierung bzw. die Position der zweiten Markierung während des ersten Zeitintervalls;
automatisches Berechnen einer Registrierung zwischen den Positionen der ersten und zweite Markierung und den Positionen der ersten und zweiten Lokalisierungselemente; und Überlagern einer Position eines medizinischen Instruments auf eine Bildanzeige des dynamischen Körpers (B) aus dem ersten Zeitintervall.

15. Das prozessorlesbare Medium gemäß Anspruch 9, wobei das Kleidungsstück (20) sowohl während des ersten Zeitintervalls als auch des zweiten Zeitintervalls in einer relativ zu dem dynamischen Körper fixierten Position mit dem dynamischen Körper gekoppelt (B) ist.

16. Das prozessorlesbare Medium gemäß Ansprüchen 8 oder 9, wobei der Code auch Anweisungen darstellt, um den Prozessor (30) zu veranlassen, des Weiteren folgende Verfahren durchzuführen:
Bereitstellen eines Navigationswegs für ein medizinisches Instrument, das basierend auf dem identifizierten Bild gelenkt werden soll.

17. Das prozessorlesbare Medium gemäß Ansprüchen 8 oder 9, wobei die erste Markierung (22) und die zweite Markierung (22) in einer relativ zu dem dynamischen Körper fixierten Position mit dem dynamischen Körper gekoppelt (B) sind, sich aber relativ zueinander bewegen können, wenn der dynamische Körper sich durch eine Bewegungsbahn bewegt, wobei das erste Lokalisierungselement (24) und das zweite Lokalisierungselement (24) in einer relativ zu dem dynamischen Körper fixierten Position mit dem dynamischen Körper gekoppelt sind, sich aber relativ zueinander bewegen können, wenn der dynamische Körper sich durch eine Bewegungsbahn bewegt.

18. Das prozessorlesbare Medium gemäß Ansprüchen 8 oder 9, wobei das Empfangen eines Indikators einer Position einer ersten Markierung (22) und eines Indikators einer Position der zweiten Markierung (22) das Empfangen einer Voxel-Koordinate der ersten Markierung an dem ersten Ort und einer Voxel-Koordinate der zweiten Markierung an dem zweiten Ort umfasst.

19. Das prozessorlesbare Mediumverfahren gemäß Anspruch 9, wobei das Kleidungsstück (20) eine im Wesentlichen planare Konfiguration aufweist.

20. Das prozessorlesbare Medium gemäß Ansprüchen 8 oder 9, wobei das Empfangen von Bilddaten das Empfangen von Bilddaten von einem Computertomographiegerät umfasst.

21. Das prozessorlesbare Medium gemäß Ansprüchen 8 oder 9, wobei das Empfangen von Bilddaten das Empfangen von Bilddaten von einem Computertomographie-Positronenemissionstomographiegerät umfasst, das einen fusionierten Computertomographie-Positronenemissionstomographie-Bilddatensatz produziert.

22. Das prozessorlesbare Medium gemäß Ansprüchen 8 oder 9, wobei das Empfangen von Bilddaten das Empfangen von Bilddaten von einem Magnetresonanztomographiegerät umfasst.

23. Das prozessorlesbare Medium gemäß Ansprüchen 8 oder 9, wobei der Code auch Anweisungen darstellt, um den Prozessor (30) zu veranlassen, des Weiteren folgende Verfahren durchzuführen:
kontinuierliches Empfangen der mit einer Position des ersten Lokalisierungselements (24) assoziierten Daten und der mit einer Position des zweiten Lokalisierungselements (24) assoziierten Daten während des zweiten Zeitintervalls und kontinuierliches Aufzeichnen der mit einer Position des ersten Lokalisierungselements assoziierten Daten und der mit einer Position des zweiten Lokalisierungselements assoziierten Daten während des zweiten Zeitintervalls.

24. Das prozessorlesbare Medium gemäß Anspruch 9, wobei der Code auch Anweisungen darstellt, um den Prozessor (30) zu veranlassen, des Weiteren folgende Verfahren durchzuführen:
Empfangen, während des ersten Zeitintervalls, eines Indikators einer Position einer dritten Markierung (22) auf dem Kleidungsstück und einer Position einer vierten Markierung (22) auf dem Kleidungsstück (20), wobei die dritte Markierung an einem dritten Ort mit dem Kleidungsstück gekoppelt ist und die vierte Markierung an einem vierten Ort mit dem Kleidungsstück gekoppelt ist, wobei die dritte und vierte Markierung sich während des ersten Zeitintervalls in ihrer Ausrichtung zueinander ändern;
Bestimmen eines Abstands zwischen der Position der dritten Markierung und der Position der vierten Markierung;
Empfangen, während des zweiten Zeitintervalls, von Daten, die mit einer Position eines dritten Lokalisierungselements (24) assoziiert sind, das an dem dritten Ort mit dem Kleidungsstück gekoppelt ist, und von Daten, die mit einer Position eines vierten Lokalisierungselements (24) assoziiert sind, das an dem vierten Ort mit dem Kleidungsstück gekoppelt ist, wobei das dritte und vierte Lokalisierungselement sich während des zweiten Zeitintervalls in ihrer Ausrichtung zueinander ändern;
Bestimmen eines Abstands zwischen dem dritten Lokalisierungselement und dem vierten Lokalisierungselement, basierend auf den mit der Position des dritten Lokalisierungselements und der Position des zweiten Lokalisierungselements assoziierten Daten; und
Berechnen, ob der Abstand zwischen der dritten Markierung und der vierten Markierung während des ersten Zeitintervalls sich von dem Abstand zwischen dem dritten Lokalisierungselement und dem vierten Lokalisierungselement während des zweiten Zeitintervalls unterscheidet.

25. Das prozessorlesbare Medium gemäß Anspruch 9, wobei das Kleidungsstück (20) dazu ausgelegt ist, die Bewegung des dynamischen Körpers (B) mindestens teilweise einzuschränken.

26. Das prozessorlesbare Medium gemäß Ansprüchen 8 oder 9, wobei das automatische Identifizieren das automatische Segmentieren der Daten umfasst, die mit der Vielzahl von Bildern aus dem ersten Zeitintervall assoziiert sind, um eine Position der ersten Markierung (22) und eine Position der zweiten Markierung (22) zu identifizieren.

27. Das prozessorlesbare Medium gemäß Ansprüchen 8 oder 9, wobei das automatische Identifizieren das automatische Korrelieren der mit der Position der ersten Markierung (22) und der Position der zweiten Markierung (22) assoziierten Daten aus dem ersten Zeitintervall mit den mit der Position des ersten Lokalisierungselements (24) und der Position des zweiten Lokalisierungselements (24) assoziierten Daten umfasst.

28. Das prozessorlesbare Medium gemäß Ansprüchen 8 oder 9, wobei das automatische Identifizieren das automatische Registrieren der Bewegungsbahn des dynamischen Körpers (B) während des ersten Zeitintervalls mit der Bewegungsbahn des dynamischen Körpers während des zweiten Zeitintervalls umfasst.

29. Das prozessorlesbare Medium gemäß Anspruch 8 oder 9, wobei die Vielzahl von Bildern eine Vielzahl von 3-D-Bildern des dynamischen Körpers (B) im Verlauf einer Bewegungsbahn des dynamischen Körpers umfasst.

## Revendications

1. Appareil (10), comprenant:
un premier marqueur (22) couplé à un vêtement (20), à un premier emplacement, un deuxième marqueur (22) couplé au vêtement (20) à un deuxième emplacement, le vêtement étant configuré pour être couplé à un corps dynamique (B) dans une position fixe par rapport au corps dynamique (B),
**caractérisé en ce que** le premier et le deuxième marqueur changent d'orientation l'un par rapport à l'autre durant un parcours de mouvement du corps dynamique et dans lequel le premier et le deuxième marqueur sont radio-opaques;
un premier élément de localisation (24) est couplé au vêtement à proximité de l'emplacement du premier marqueur ; et
un deuxième élément de localisation (24) est couplé au vêtement à proximité de l'emplacement du deuxième marqueur, le premier élément de localisation et le deuxième élément de localisation comprenant des bobines électromagnétiques et chacun étant couplé à un dispositif récepteur (34) et étant configuré pour envoyer simultanément au dispositif récepteur des données de position associées à une pluralité de positions dans un espace tridimensionnel du premier élément de localisation et des données de position associées à une pluralité de positions dans un espace tridimensionnel du deuxième élément de localisation durant le parcours de mouvement du corps dynamique, où le premier et le deuxième élément de localisation changent d'orientation l'un par rapport à l'autre durant le parcours de mouvement du corps dynamique (B),
dans lequel le dispositif récepteur détermine une distance (d1 - d6) entre la position du premier élément de localisation et la position du deuxième élément de localisation sur la base des données de position pour chacun d'une pluralité de moments de temps durant le parcours de mouvement du corps dynamique.

2. Appareil selon la revendication 1, où le vêtement (20) est sensiblement plan.

3. Appareil selon la revendication 1, où le vêtement (20) a une configuration de manchon.

4. Appareil selon la revendication 1, où le vêtement (20) est configuré pour être couplé à la partie supérieure du torse d'un patient.

5. Appareil selon la revendication 1, où le vêtement (20) est configuré pour gêner partiellement au moins le mouvement du corps dynamique (B).

6. Appareil selon la revendication 1, où le vêtement (20) est configuré pour être couplé au corps dynamique (B) en utilisant au moins l'un d'entre un adhésif, une ou plusieurs sangles, un ou plusieurs dispositifs d'attache auto-aggripants ou un ou plusieurs boutons-pression.

7. Appareil selon la revendication 1, où le vêtement (20) est sous forme de patch configuré pour être disposé sur le corps d'un patient.

8. Support lisible par processeur stockant du code représentant des instructions pour faire qu'un processeur (30) exécute un procédé, le procédé comprenant :
recevoir durant un premier intervalle de temps des données d'images associées à une pluralité d'images d'un corps dynamique (B), la pluralité d'images comprenant une indication d'une position d'un premier marqueur (22) couplé au corps dynamique et d'une position d'un deuxième marqueur (22) couplé au corps dynamique, le premier marqueur étant couplé au corps dynamique à un premier emplacement, le deuxième marqueur étant couplé au corps dynamique à un deuxième emplacement, le premier intervalle de temps associé à un premier parcours de mouvement du corps dynamique, où le premier et le deuxième marqueur changent d'orientation l'un par rapport à l'autre durant le premier parcours de mouvement du corps dynamique et où le premier et le deuxième marqueur sont radio-opaques;
recevoir durant un deuxième intervalle de temps après le premier intervalle de temps des données associées à une position d'un premier élément de localisation (24) par rapport au corps dynamique, et des données associées à une position d'un deuxième élément de localisation (24) par rapport au corps dynamique, le premier élément de localisation étant couplé au corps dynamique à proximité du premier emplacement, le deuxième élément de localisation étant couplé au corps dynamique à proximité du deuxième emplacement, le deuxième intervalle de temps étant associé à un deuxième parcours de mouvement du corps dynamique, où le premier et le deuxième élément de localisation changent d'orientation l'un par rapport à l'autre durant le deuxième parcours de mouvement du corps dynamique et où le premier et le deuxième élément de localisation sont des bobines électromagnétiques ;
déterminer une distance (D1 - D6) entre la position du premier élément de localisation et la position du deuxième élément de localisation sur la base des données de position pour chacun d'une pluralité de moments de temps durant le parcours de mouvement du corps dynamique ; et
identifier automatiquement durant le deuxième intervalle de temps une image de la pluralité d'images associées à une position du premier marqueur qui est sensiblement la même que la position du premier élément de localisation par rapport au corps dynamique et à une position du deuxième marqueur qui est sensiblement la même que la position du deuxième élément de localisation par rapport au corps dynamique.

9. Support lisible par processeur selon la revendication 8, où le premier marqueur (22) est couplé à un vêtement (20), le vêtement étant couplé au corps dynamique (B) et le deuxième marqueur (22) étant couplé au vêtement, le premier marqueur étant couplé au vêtement au premier emplacement, le deuxième marqueur étant couplé au vêtement au deuxième emplacement ;
le premier élément de localisation (24) étant couplé au vêtement au premier emplacement, le deuxième élément de localisation (24) étant couplé au vêtement au deuxième emplacement.

10. Support lisible par processeur selon les revendications 8 ou 9, où le fait d'identifier automatiquement comprend identifier une position du premier marqueur (22) sur la base d'un aspect du premier marqueur dans l'image identifiée et identifier une position du deuxième marqueur (22) sur la base d'un aspect du deuxième marqueur dans l'image identifiée.

11. Support lisible par processeur selon les revendications 8 ou 9, le code représentant aussi des instructions pour faire que le processeur (30) exécute en outre le procédé consistant à :
recevoir durant le deuxième intervalle de temps des données associées à une position d'un troisième élément de localisation (24) par rapport au corps dynamique (B), le troisième élément de localisation étant couplé au corps dynamique ;
projeter la position du troisième élément de localisation sur la base des données associées à la pluralité d'images ; et
déterminer qu'un troisième marqueur (22) soit l'un d'entre existe ou n'existe pas dans une image de la pluralité d'images du premier intervalle de temps à sensiblement la même position que le troisième élément de localisation.

12. Support lisible par processeur selon les revendications 8 ou 9, où le fait d'identifier automatiquement comprend mettre en corrélation la position du premier élément de localisation (24) durant le deuxième intervalle de temps avec la position du premier marqueur (22) dans une image de la pluralité d'images.

13. Support lisible par processeur selon les revendications 8 ou 9, le code représentant aussi des instructions pour faire que le processeur (30) exécute en outre le procédé consistant à :
enregistrer automatiquement le parcours de mouvement du corps dynamique (B) durant le deuxième intervalle de temps avec le parcours de mouvement du corps dynamique durant le premier intervalle de temps sur la base du fait d'identifier automatiquement.

14. Support lisible par processeur selon les revendications 8 ou 9,
où le fait d'identifier automatiquement comprend déterminer une mesure entre la position du premier marqueur (22) et la position du deuxième marqueur (22) associées à la pluralité d'images durant le premier intervalle de temps en utilisant une fonction-coût sélectionnée ;
appliquer la fonction-coût sélectionnée à la position du premier élément de localisation (24) et à la position du deuxième élément de localisation (24) durant le deuxième intervalle de temps ;
déterminer si la position du premier élément de localisation et si la position du deuxième élément de localisation durant le deuxième intervalle de temps est sensiblement la même que la position du premier marqueur et que la position du deuxième marqueur, respectivement, durant le premier intervalle de temps ;
calculer automatiquement un enregistrement entre les positions du premier et du deuxième marqueur, et les positions du premier et du deuxième élément de localisation ; et superposer une position d'un instrument médical sur un affichage d'image du corps dynamique (B) du premier intervalle de temps.

15. Support lisible par processeur selon la revendication 9, où le vêtement (20) est couplé au corps dynamique (B) dans une position fixe par rapport au corps dynamique durant le premier intervalle de temps et le deuxième intervalle de temps tous les deux.

16. Support lisible par processeur selon les revendications 8 et 9, le code représentant aussi des instructions pour faire que le processeur (30) exécute en outre le procédé consistant à :
fournir un chemin de navigation pour qu'un instrument médical soit dirigé sur la base de l'image identifiée.

17. Support lisible par processeur selon les revendications 8 ou 9, où le premier marqueur (22) et le deuxième marqueur (22) sont couplés au corps dynamique (B) dans une position fixe par rapport au corps dynamique, mais peuvent se déplacer l'un par rapport à l'autre lorsque le corps dynamique se déplace le long d'un parcours de mouvement, le premier élément de localisation (24) et le deuxième élément de localisation (24) sont couplés au corps dynamique à une position fixe par rapport au corps dynamique, mais peuvent se déplacer l'un par rapport à l'autre lorsque le corps dynamique se déplace le long d'un parcours de mouvement.

18. Support lisible par processeur selon les revendications 8 ou 9, où le fait de recevoir une indication d'une position d'un premier marqueur (22) et une indication d'une position du deuxième marqueur (22) comprend recevoir une coordonnée voxel du premier marqueur au premier emplacement et une coordonnée voxel du deuxième marqueur au deuxième emplacement.

19. Procédé à support lisible par processeur selon la revendication 9, où le vêtement (20) a une configuration sensiblement plane.

20. Support lisible par processeur selon les revendications 8 ou 9, où le fait de recevoir des données d'images comprend recevoir des données d'images d'un dispositif de tomographie assistée par ordinateur.

21. Support lisible par processeur selon les revendications 8 ou 9, où le fait de recevoir des données d'images comprend recevoir des données d'images d'un dispositif de tomographie assistée par ordinateur-tomographie par émission de positrons qui produit un ensemble de données d'images de tomographie assistée par ordinateur-tomographie par émission de positrons fusionnées.

22. Support lisible par processeur selon les revendications 8 ou 9, où le fait de recevoir des données d'images comprend recevoir des données d'images d'un dispositif d'imagerie par résonance magnétique.

23. Support lisible par processeur selon les revendications 8 ou 9, le code représentant aussi des instructions pour faire que le processeur (30) exécute en outre le procédé consistant à :
recevoir les données associées à une position du premier élément de localisation (24) et les données associées à une position du deuxième élément de localisation (24) continuellement durant le deuxième intervalle de temps et enregistrer les données associées à une position du premier élément de localisation et les données associées à une position du deuxième élément de localisation continuellement durant le deuxième intervalle de temps.

24. Support lisible par processeur selon la revendication 9, le code représentant aussi des instructions pour faire que le processeur (30) exécute en outre le procédé consistant à :
recevoir durant le premier intervalle de temps une indication d'une position d'un troisième marqueur (22) sur le vêtement et d'une position d'un quatrième marqueur (22) sur le vêtement (20), le troisième marqueur étant couplé au vêtement à un troisième emplacement et le quatrième marqueur étant couplé au vêtement à un quatrième emplacement, où le troisième et le quatrième marqueur changent d'orientation l'un par rapport à l'autre durant le premier intervalle de temps ;
déterminer une distance entre la position du troisième marqueur et la position du quatrième marqueur ;
recevoir durant le deuxième intervalle de temps des données associées à une position d'un troisième élément de localisation (24) couplé au vêtement au troisième emplacement et des données associées à une position d'un quatrième élément de localisation (24) couplé au vêtement au quatrième emplacement, où le troisième et le quatrième élément de localisation changent d'orientation l'un par rapport à l'autre durant le deuxième intervalle de temps ;
déterminer une distance entre le troisième élément de localisation et le quatrième élément de localisation sur la base des données associées à la position du troisième élément de localisation et à la position du deuxième élément de localisation ; et
calculer si la distance entre le troisième marqueur et le quatrième marqueur durant le premier intervalle de temps est différente de la distance entre le troisième élément de localisation et le quatrième élément de localisation durant le deuxième intervalle de temps.

25. Support lisible par processeur selon la revendication 9, où le vêtement (20) est configuré pour gêner partiellement au moins le mouvement du corps dynamique (B).

26. Support lisible par processeur selon les revendications 8 ou 9, où le fait d'identifier automatiquement comprend segmenter automatiquement les données associées à la pluralité d'images du premier intervalle de temps afin d'identifier une position du premier marqueur (22) et une position du deuxième marqueur (22).

27. Support lisible par processeur selon les revendications 8 ou 9, où le fait d'identifier automatiquement comprend mettre automatiquement en corrélation les données associées à la position du premier marqueur (22) et à la position du deuxième marqueur (22) du premier intervalle de temps avec les données associées à la position du premier élément de localisation (24) et à la position du deuxième élément de localisation (24).

28. Support lisible par processeur selon les revendications 8 ou 9, où le fait d'identifier automatiquement comprend enregistrer automatiquement le parcours de mouvement du corps dynamique (B) durant le premier intervalle de temps avec le parcours de mouvement du corps dynamique durant le deuxième intervalle de temps.

29. Support lisible par processeur selon la revendication 8 ou 9, où la pluralité d'images comprend une pluralité d'images 3-D du corps dynamique (B) durant un parcours de mouvement du corps dynamique.
